# EUROPEAN PATENT APPLICATION

(11) **EP 4 099 002 A1**
(43) Date of publication of application: **07.12.2022**
(21) Application number: 21747649.8
(22) Date of filing: 13.01.2021
(51) Int. Cl.: G01N 21/63, G01N 33/558, G01N 21/31, G01N 21/84

(54) **CHROMATOGRAPHIC INSPECTION APPARATUS AND CONTROL METHOD THEREOF**

(30) Priority: 30.01.2020 KR 20200011106
(71) Applicant: OSANG HEALTHCARE CO., LTD., Anyang-si, Gyeonggi-do 14040 (KR)
(72) Inventor: SHIN, Dae Sup, Seoul 01223 (KR); SHIM, Dong Whi, Incheon 21584 (KR); CHUNG, Hyun Woo, Seoul 01858 (KR); HONG, Ju Pyo, Seoul 02517 (KR); KO, Hee Young, Sokcho-si Gangwon-do 24848 (KR)
(74) Representative: AWA Sweden AB
(86) International application number: PCT/KR2021/000472
(87) International publication number: WO 2021/153928

(57) **Abstract**

The present invention relates to a chromatographic inspection apparatus of which the size is miniaturized and which is capable of optimal light emission and reading control according to the type of marker, and a control method thereof. Provided is a chromatographic inspection apparatus, according to one aspect of the present invention, comprising: a main body; a cartridge tray on which an immunochromatographic test cartridge is seated, and which is provided so as to be accommodated in the main body or to be withdrawn to the outside of the main body; a tray driving unit for moving the cartridge tray; an image sensing unit for recognizing a code expression provided on a surface of the cartridge or excitation light generated from the cartridge; a first light source illuminating the code expression provided on the surface of the cartridge; a second light source illuminating a window of the cartridge to generate the excitation light in the cartridge; and a control unit for controlling the tray driving unit, the first light source, and the second light source, and processing information acquired from the image sensing unit.

## Description

### [Technical Field]

The present invention relates to a chromatographic inspection apparatus and a control method thereof, and more specifically to a chromatographic inspection apparatus of which the size is miniaturized and which is capable of optimal light emission and reading control according to the type of marker, and a control method thereof.

### [Background Art]

The immune chromatography test, also known as the rapid test, is a method that can qualitatively and quantitatively test a trace amount of analyte in a short time by using an antigen-antibody reaction, and it is used in various fields such as medicine, agriculture, food, environment and the like, as well as testing for various diseases.

In this immune chromatography test, a sample such as blood, body fluid or the like is applied to a cartridge equipped with an analysis strip coated or impregnated with an antigen material that reacts with a specific antibody in the sample, and then the presence or absence of the antibody is determined through the reaction result of the analysis strip.

In addition, when the amount of antibody is small, the antigen-antibody reaction may also be weak, and in order to further maximize this, it can be identified using a fluorescence reaction or the like.

A chromatographic inspection apparatus is provided to perform an immunochromatographic test through the fluorescence reaction.

The chromatographic inspection apparatus can determine the presence and extent of antigens such as whether infection occurred by analyzing the fluorescence reaction (excitation light) generated by irradiating light to an analysis strip to which the sample is applied.

Meanwhile, such a chromatographic test method is often manufactured based on biochemical materials such as immunological substances and the like, and due to the characteristics of the materials of these quantitative measurement biosensors, different signal reproducibility is obtained for each manufacturing lot, and accordingly, a code expression which is suitable for each lot is provided such that the deviation of signals between the lots can be corrected.

In general, although the code expression for each marker such as the biosensor applied to the cartridge is utilized for measurement by using an information medium such as a chip or RFID, it is inconvenient to separately recognize the corresponding code and apply the change whenever the lot of the biosensor is changed. In order to solve this inconvenience, efforts were made to recognize the information by attaching a barcode corresponding to the biosensor applied to each cartridge, but to this end, a separate sensor must be built into the chromatographic inspection apparatus, which may lead to an enlargement of the apparatus and an increase in the manufacturing costs.

### [Disclosure]

### [Technical Problem]

The present invention has been devised to solve the above problems, and it is an object of the present invention to provide a chromatographic inspection apparatus, which is capable of recognizing information on a cartridge while minimizing additionally provided components.

In addition, it is an object of the present invention to provide a chromatographic inspection apparatus, which is capable of recognizing the type of marker such as a biosensor applied to the cartridge and operating optimally for the type of the corresponding marker.

The problems of the present invention are not limited to the problems mentioned above, and other problems that are not mentioned will be clearly understood by those skilled in the art from the following description.

### [Technical Solution]

In order to solve the aforementioned problems, according to an aspect of the present invention, provided is a chromatographic inspection apparatus, including a main body, a cartridge tray on which an immunochromatographic test cartridge is seated, and which is provided so as to be accommodated in the main body or to be withdrawn to the outside of the main body, a tray driving unit for moving the cartridge tray; an image sensing unit for recognizing a code expression provided on a surface of the cartridge or excitation light generated from the cartridge, a first light source illuminating the code expression provided on the surface of the cartridge, a second light source illuminating a window of the cartridge to generate the excitation light in the cartridge, and a control unit for controlling the tray driving unit, the first light source and the second light source, and processing information acquired from the image sensing unit.

The first light source may emit light in a wavelength band different from that of the second light source.

The image sensing unit may include an image sensor for sensing incident light; and a filter for blocking light other than the wavelength band of the excitation light from being incident on the image sensor.

The first light source may emit light in a wavelength band that passes through the filter.

The second light source may be installed on both sides of the image sensor and irradiate obliquely from both sides of the window of the cartridge.

The chromatographic inspection apparatus may further include a fixing part for elastically fixing the seated cartridge.

The fixing part may include a ball plunger provided in the cartridge tray and elastically supported, and elastically inserted into a fixing groove formed in the cartridge.

A calibration window, which is formed in the cartridge tray and through which the light of the second light source passes, may be further formed.

The chromatographic inspection apparatus may further include a position sensor for recognizing the position of the cartridge.

The position sensor may recognize a position where the code expression of the cartridge is located below the image sensor and a position where the window of the cartridge is located below the image sensor.

The chromatographic inspection apparatus may further include a cartridge recognition sensor provided in the cartridge tray to recognize whether the cartridge is seated.

Meanwhile, according to another aspect of the present invention, provided is a method for controlling a chromatographic inspection apparatus, which may include a cartridge seating step for seating a cartridge in a cartridge tray, a code expression recognizing step for recognizing a code expression of the cartridge seated in the cartridge tray, a blood injection step for dropping a sample into the cartridge, an excitation position moving step for moving the cartridge on which the blood injection has been completed to an excitation position for reading excitation light, an excitation light source irradiating step for irradiating an excitation light source for emitting excitation light to the cartridge according to the recognition result of the code expression recognizing step, and an excitation light measuring step for recognizing and measuring excitation light emitted according to the irradiation of the excitation light source.

The type of marker of the cartridge may be recognized according to the recognition result of the code expression recognizing step, and the emission of the excitation light source may be controlled to correspond to the type of the marker.

The method may further include a code expression moving step for moving the cartridge seated in the cartridge tray for the recognition of code expression.

### [Advantageous Effects]

According to the chromatographic inspection apparatus and the control method thereof according to the present invention, the following effects are obtained.

First, it is possible to automatically recognize the code expression of the cartridge and control and measure corresponding to the code expression, thereby improving accuracy and convenience.

Second, since the code expression of the cartridge can be automatically recognized while minimizing additional components, it is possible to reduce the size while reducing the cost.

Third, the wavelength band of the light source may change over time, and the excitation light generated from the cartridge may also change accordingly, and since the calibration hole is provided, it is possible to adjust the wavelength band of the light source or adjust the image sensor and the control unit for receiving the excitation light, and thus, there is an effect that accuracy can be improved through correction.

The effects of the present invention are not limited to the effects mentioned above, and other effects that are not mentioned will be clearly understood by those skilled in the art from the description of the claims.

### [Description of Drawings]

The summary set forth above as well as the detailed description of the preferred exemplary embodiments of the present application set forth below may be better understood when read in conjunction with the accompanying drawings. For the purpose of illustrating the present invention, preferred exemplary embodiments are illustrated in the drawings. It should be understood, however, that the present application is not limited to the precise arrangements and instrumentalities illustrated therein.
FIG. 1 is a perspective view illustrating the chromatographic inspection apparatus according to an exemplary embodiment of the present invention;
FIG. 2 is a perspective view illustrating the inside of the chromatographic inspection apparatus of FIG. 1;
FIG. 3 is a perspective view illustrating the cartridge tray and the tray driving unit of the chromatographic inspection apparatus of FIG. 1;
FIG. 4 is a perspective view illustrating a cartridge;
FIG. 5 is a perspective view illustrating an image sensing unit, a first light source and a second light source;
FIG. 6 is a cross-sectional view of FIG. 5;
FIGS. 7 and 8 are views illustrating a position sensor and a cartridge recognition sensor; and
FIG. 9 is a flowchart illustrating an exemplary embodiment of the method for controlling a chromatographic inspection apparatus of the present invention.

### [Modes of the Invention]

Hereinafter, preferred exemplary embodiments of the present invention in which the objects of the present invention may be specifically implemented will be described with reference to the accompanying drawings. In describing the present exemplary embodiments, the same names and the same reference numerals are used for the same components, and an additional description thereof will be omitted.

Hereinafter, an exemplary embodiment of the chromatographic inspection apparatus 100 of the present invention will be described.

In the description of the chromatographic inspection apparatus 100 of the present exemplary embodiment, the use of blood or body fluid as an antibody sample will be described as an example. However, the present invention is not necessarily limited thereto, and various samples other than blood or body fluid may be applied.

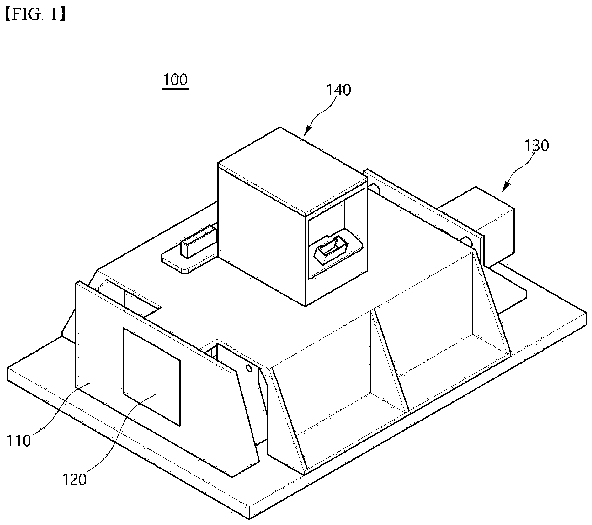

As illustrated in FIGS. 1 to 5, the chromatographic inspection apparatus 100 according to the present exemplary embodiment may include a main body 110, a cartridge tray 120, a tray driving unit 130, an image sensing unit 140, a first light source 150, a second light source 160 and a control unit 180.

The main body 110 forms an external shape, and a space for immunochromatographic analysis and a space in which parts are provided may be formed therein.

The cartridge tray 120 may be movably provided to be accommodated inside the main body 110 or to be withdrawn to the outside of the main body 110.

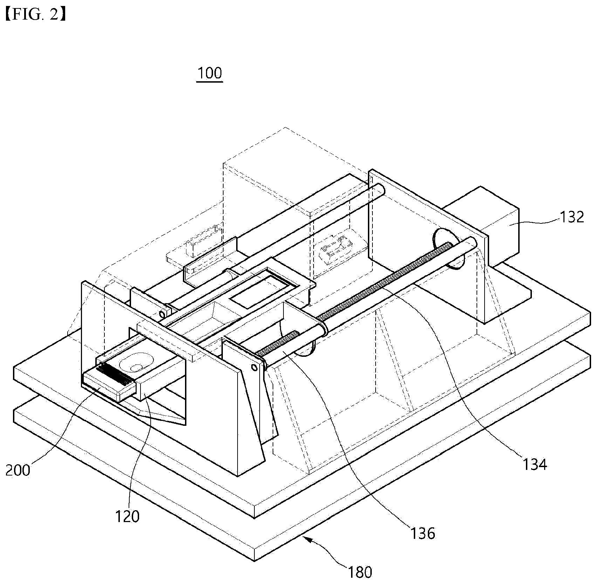

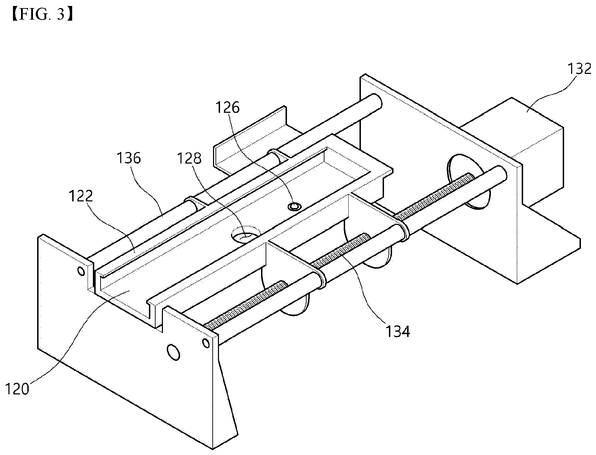

Meanwhile, a tray driving unit 130 for driving the cartridge tray 120 may be provided. As illustrated in FIGS. 2 and 3, the tray driving unit 130 may include a motor 132 capable of rotating in the forward direction and rotating in the reverse direction under the control of the control unit 180, and a screw 134 rotated by the motor 132 and having a longitudinal direction parallel to the moving direction of the cartridge tray 120, and having a screw thread formed on the outer peripheral surface in the longitudinal direction. In addition, the screw 134 may be engaged with the cartridge tray 120 such that the cartridge tray 120 slides in one or the other direction according to the rotation of the screw 134. In addition, a guide 136 for guiding the movement of the cartridge tray 120 may be provided.

The cartridge tray 120 may be provided such that the immunochromatography cartridge 200 (hereinafter, referred to as 'a cartridge 200' for the convenience of description) having an assay strip is inserted and mounted therein.

In addition, the rail 122 may be provided such that the cartridge 200 may be mounted in a sliding manner for more accurate and easy mounting. That is, the cartridge tray 120 may be provided with a rail 122 slidably coupled to the side surface of the cartridge 200 such that the cartridge 200 is mounted while being drawn in in the horizontal direction from the front surface. In this case, the rail 122 may be provided so as not to interfere with the upper surface of the cartridge 200 mounted on the cartridge tray 120.

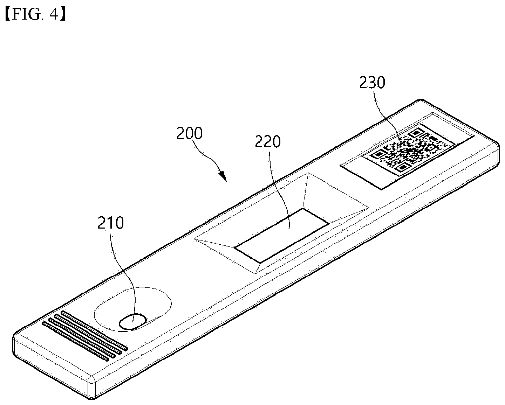

As illustrated in FIG. 4, the cartridge 200 may include an immune strip inside a housing that forms an external shape.

In addition, an injection hole 210 for applying a sample such as blood or body fluid to the immune strip may be formed in the housing.

In addition, a window 220 may be formed to confirm the reaction of the immune strip to which the sample is applied or to confirm that the excitation light is emitted.

In addition, a code expression 230 in which information such as the type of marker such as a biosensor applied to a lot of the cartridge 200 or an immune strip may be printed or formed on one side of the housing.

The code expression 230 may be formed of a barcode, QR code or the like, but the present invention is not limited to the form of the code expression 230.

Meanwhile, the image sensing unit 140 recognizes the code expression 230 of the cartridge 200, and is provided to confirm the reaction appearing in the immune strip of the cartridge 200 through a window 220.

The image sensing unit 140 may include an image sensor 142 for sensing image information and a filter 144 which is a component for filtering light received by the image sensor 142 so as not to pass through a specific wavelength band.

In addition, the image sensing unit 140 may include a lens group 146 for refracting the light received by the image sensor 142.

The filter 144 may be provided between the lens group 146 and the image sensor 142. Alternatively, the filter 144 may be provided on the front surface of the lens group 146.

In the present exemplary embodiment, the filter 144 is described as an example where the filter 144 is manufactured in a way that is fitted in the form of a cap on the lens group 146, but the present invention is not limited thereto, and the position of the filter 144 may be installed at a suitable position according to the needs of the manufacturer.

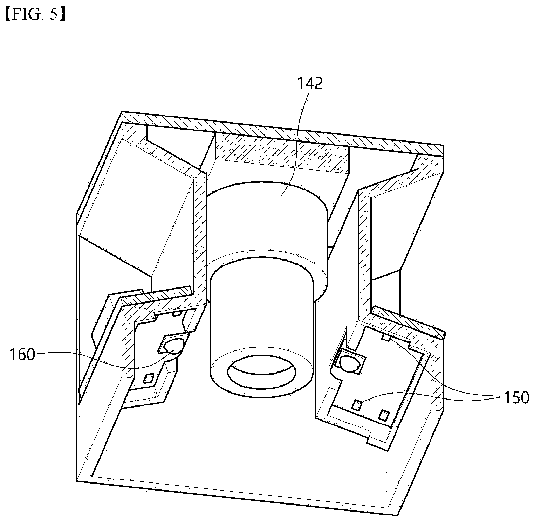

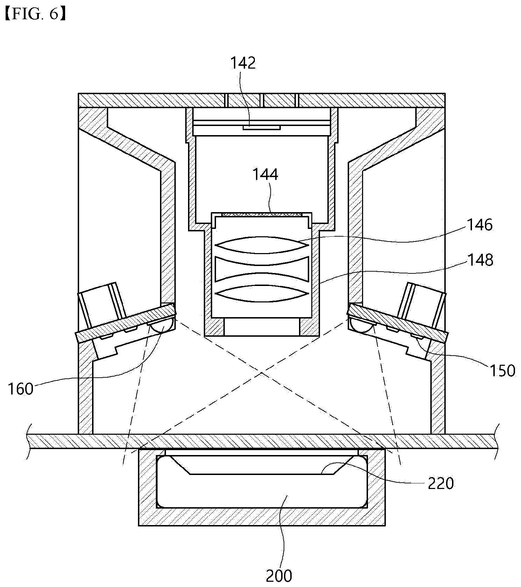

Meanwhile, as illustrated in FIGS. 5 and 6, in order for the imaging sensing unit 140 to recognize the code expression 230 of the cartridge 200, the first light source 150 may be provided to illuminate a portion where the code expression 230 of the cartridge 200 is formed.

As illustrated in FIGS. 5 and 6, the first light source 150 is provided around the image sensor 142, and may be provided to illuminate a portion where the code expression 230 of the cartridge 200 is formed. The first light source 150 may be provided as a light emitting diode (LED), and if necessary, other lighting means other than the LED may be provided.

In addition, as illustrated in FIGS. 5 and 6, a second light source 160 may be provided. The second light source 160 is provided around the image sensor 142 and may be provided to illuminate a portion of the window 220 of the cartridge 200.

The second light source 160 is an excitation light source for inducing an excitation light reaction of the immune strip of the cartridge 200 that has reacted with the antibody of the sample, and may be provided to illuminate the window 220 of the cartridge 200. In this case, an LED may be applied to the second light source 160 like the first light source 150, but the present invention is not limited thereto, and other types of light sources may be applied as needed.

Meanwhile, the first light source 150 and the second light source 160 may be provided to emit light in different wavelength bands.

In this case, the excitation light generated from the window 220 of the cartridge 200 should be incident to the image sensor 142, not the light from the second light source 160, and to this end, this is because a filter 144 is provided to block light other than the wavelength band of the excitation light generated in the window 220 from being incident on the image sensor.

Accordingly, the light of the second light source 160 is not directly incident on the image sensor, and the excitation light of the cartridge 200 generated by being excited by the second light source 160 passes through the filter 144, and thus, light may be received by the image sensor 142.

In this case, since the light emitted from the first light source 150 must be received by the image sensor 142 after being reflected by the code expression 230, the light emitted from the first light source 150 may be light in a wavelength band capable of passing through the filter 144.

Meanwhile, the second light source 160 may be provided on both sides of the image sensor 142. That is, a plurality of second light sources 160 may be provided to be symmetrically and obliquely illuminated from the side toward the window 220 of the cartridge 200.

The reason why the plurality of second light sources 160 are symmetrically and obliquely illuminated from the side is to uniformly irradiate an excitation light source without a shade on the analysis strip exposed by the window 220.

To this end, as illustrated in FIGS. 4 and 6, the edge of the window 220 of the housing of the cartridge 200 may be formed to be obliquely inclined to minimize the step difference.

In addition, the control unit 180 is a component for controlling the tray driving unit 130, the image sensing unit 140, the first light source 150 and the second light source 160 described above, and it may be provided inside the main body 110. In addition, the control unit 180 may be provided to analyze information acquired through the image sensing unit 140 to determine whether the antigen is present in the sample and the degree thereof.

In addition, the control unit 180 may control the second light source 160 and the image sensing unit 140 according to the lot and type of the cartridge 200 recognized through the code expression 230.

That is, as information collected through the recognition of the code expression 230, optimal control corresponding to the corresponding lot and the type of the biomarker is performed through the lot of the cartridge 200 and the type of the biomarker.

For example, the light emission of the second light source 160 is controlled depending on whether the type of the biomarker of the corresponding cartridge 200 recognized through the code expression 230 is a time resolved fluorescence (TRF) method or a quantum dot method, and accordingly, the analysis pattern of the excitation light recognized by the image sensor 142 may be optimally controlled.

Meanwhile, a fixing part for elastically fixing the cartridge 200 mounted on the cartridge tray 120 may be further included.

The fixing part may guide the cartridge 200 to the correct position by elastically fixing the cartridge 200 to the correct position of the cartridge tray 120, and may prevent the mounted cartridge 200 from being moved from the correct position by an external force.

As illustrated in FIG. 3, the fixing part may include a ball plunger 126. The ball plunger 126 is provided in the cartridge tray 120, and it is elastically supported by a spring (not illustrated) or the like such that a part of the sphere is exposed to the outside.

In addition, a fixing groove (not illustrated) into which the ball plunger 126 can be inserted may be formed in the cartridge 200.

Therefore, when the cartridge 200 is placed in the correct position while the cartridge 200 is pushed into the cartridge tray 120, the ball plunger 126 is inserted into the fixing groove, whereby the cartridge 200 may be fixed in the correct position.

In addition, with the vibration and sound generated when the ball plunger 126 is inserted into the fixing groove, the user can know that the installation of the cartridge 200 has been completed in the correct position.

Meanwhile, the second light source 160 may deteriorate in its color temperature, luminance and light quantity over time. The deterioration of the second light source 160 may also affect the excitation light generated from the cartridge 200, and as time goes by, it is necessary to check and adjust the color temperature, luminance and light quantity of the second light source 160 at regular intervals.

However, the second light source 160 is located in a dark room such that external light is not introduced, and accordingly, a separate structure for checking the color temperature, luminance and light quantity of the second light source 160 may be required.

Therefore, according to the chromatographic inspection apparatus 100 of the present exemplary embodiment, as illustrated in FIG. 3, a calibration window 128, through which the light of the second light source 160 can pass, may be formed in the cartridge tray 120.

The calibration window 128 may be formed in the cartridge tray 120 at a position corresponding to a direct lower portion of the second light source 160 such that the light of the second light source 160 may be transmitted when the cartridge tray 120 is inserted into the body 110.

The calibration window 128 may be opened or formed of a transparent material to allow light from the second light source 160 to pass therethrough.

That is, the calibration window 128 is covered by the cartridge 200 when used normally, and when the calibration operation of the second light source 160 is required, it may be formed such that the light of the second light source 160 may be transmitted in a state where the cartridge 200 is not mounted.

In addition, a separate calibration sensor (not illustrated) capable of receiving light from the second light source 160 may be provided below the calibration window 128 or a space in which equipment for separate calibration can be located may be formed.

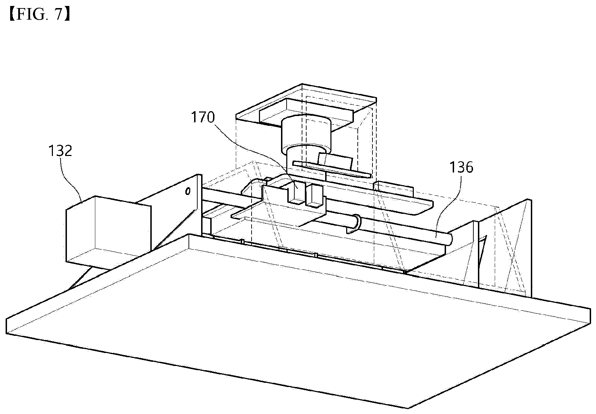

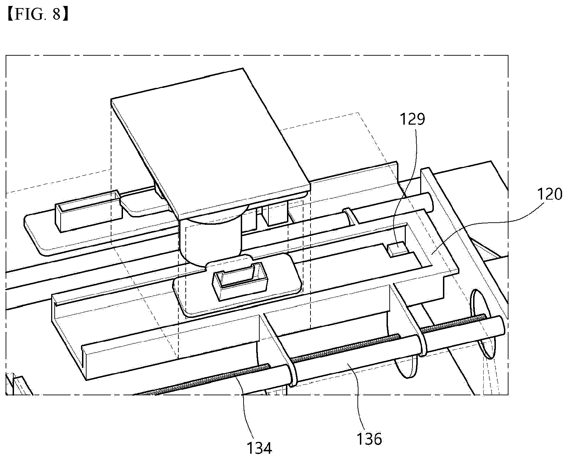

In addition, as illustrated in FIGS. 7 and 8, a cartridge recognition sensor 129 for sensing whether the cartridge 200 is mounted on the cartridge tray 120 and a position detection sensor 170 for sensing at what position the cartridge 200 or the cartridge tray 120 is located may be provided.

The position detection sensor 170 detects a position where the code expression 230 is located below the image sensor 142 such that the image sensor 142 may recognize the code expression 230, and in addition, a position at which the window 220 is positioned below the image sensor 142 may be detected such that the image sensor 142 may recognize the excitation light of the cartridge 200.

The control unit 180 may recognize whether the cartridge 200 is mounted and the current position of the cartridge 200 through the information detected by the cartridge recognition sensor 129 and the position detection sensor 170. The control unit 180 may be provided inside the body 110 or may be provided in the form of a separate circuit board on the outside of the lower side of the body 110.

Hereinafter, an exemplary embodiment of the method for controlling the chromatographic inspection apparatus 100 of the present invention will be described.

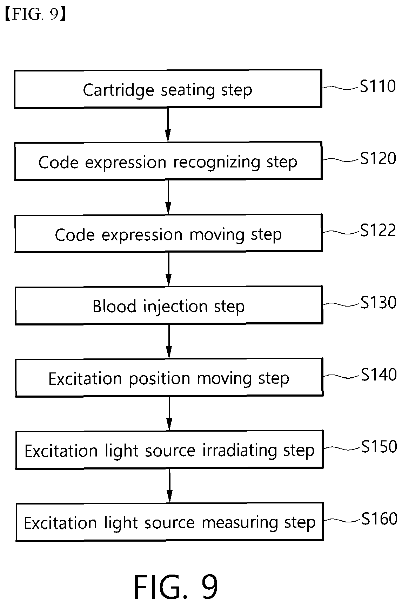

As illustrated in FIG. 9, the method for controlling the chromatographic inspection apparatus 100 according to the present exemplary embodiment may include a cartridge seating step (S110), a code expression recognizing step (S120), a blood injection step (S130), an excitation position moving step (S140), an excitation light source irradiation step (S150) and an excitation light measuring step (S160).

The cartridge seating step (S110) is a step in which the cartridge 200 is mounted on the cartridge tray 120. The seated cartridge 200 may be fixed through the aforementioned fixing part, and it may be recognized that the cartridge 200 is mounted through the cartridge recognition sensor 129.

In this case, in the cartridge 200 seated on the cartridge tray 120 in the cartridge seating step (S110), a portion where the code expression 230 is formed may be located directly below the image sensor 142.

After the cartridge seating step (S110), the code expression recognizing step (S120) may be performed. In the code expression recognizing step (S130), the first light source 150 may be operated to illuminate the code expression 230 of the cartridge 200, and the code expression 230 may be recognized in the imaging sensor unit 140.

Meanwhile, in general, the code expression 230 may be formed in a size included in the viewing range of the image sensor 142.

However, when the amount of information to be contained in the code expression 230 is large or the size of the code expression 230 is formed to be larger than the viewing range of the image sensor 142, the code expression moving step (S122) for moving the cartridge 200 for recognition of the code expression 230 may be further performed.

The code expression moving step (S122) may be performed during the code expression recognizing step (S 120), and may move the cartridge tray 120 such that the entire code expression 230 can enter the image sensor at least once within the viewing range.

Certainly, the code expression moving step (S122) is not necessarily performed, and even when the cartridge tray 120 is not moved, if the size of the code expression 230 is sufficiently large to fit into the viewing range of the image sensor 142, it may not be necessary to be performed.

In addition, the blood injection step (S130) of dropping a sample to the cartridge 200 may be performed.

The blood injection is to drop a sample such as blood or body fluid into the cartridge 200, and the sample may be dropped into the cartridge 200 through the injection hole 210 of the cartridge 200. In this case, in the cartridge seating step, the injection hole of the cartridge may be exposed at a place suitable for blood injection, such as the outside of the main body 110.

This blood injection step (S130) may be performed after or before the code expression recognizing step (S120).

The sample loaded onto the cartridge 200 may move to the immune strip according to the internal structure of the cartridge 200 to allow an antibody-antigen reaction to occur.

The excitation position moving step (S140) is a step of moving the cartridge 200 on which the blood injection has been completed to an excitation position for reading the excitation light. In this case, the excitation position may be a step in which the window 220 of the cartridge 200 is located directly below the image sensor 142.

The excitation light source irradiation step (S150) is a step of operating the second light source 160 to irradiate the light of the second light source 160 to the window 220 of the cartridge 200 on which the sample is loaded. The immune strip of the cartridge 200 on which the sample is loaded may react with an antigen contained in the sample, and in this case, when light is irradiated, the antibody reacted with the antigen may emit excitation light. The second light source 160 may act as an excitation light source for inducing such excitation light. Certainly, when there is no antigen in the sample, even if the immune strip is irradiated with the excitation light source, the excitation light reaction may not occur or the reference excitation light may not be emitted.

In addition, the excitation light measuring step (S160) may be performed. The excitation light measuring step (S160) is a step of recognizing and measuring the excitation light emitted according to the irradiation of the excitation light source through the image sensing unit. The control unit 180 may analyze the excitation light measured in the excitation light measuring step (S160) to determine whether the antibody is present in the specimen or the amount thereof.

In this case, the control unit 180 may control the optimum corresponding to the corresponding lot and the type of the biomarker through the lot and the type of the biomarker of the cartridge 200, as the information collected through the code expression 230 recognized in the aforementioned code expression recognizing step (S120).

For example, the light emission of the second light source 160 is controlled depending on whether the type of the biomarker of the cartridge 200 recognized through the code expression 230 is a TRF method or a quantum dot method, and accordingly it is possible to optimally control the analysis pattern of the excitation light recognized by the image sensor 142.

As described above, the preferred exemplary embodiments according to the present invention have been described, and the fact that the present invention can be embodied in other specific forms without departing from the spirit or scope of the present invention in addition to the above-described exemplary embodiments is apparent to those having ordinary skill in the art. Therefore, the above-described exemplary embodiments are to be regarded as illustrative rather than restrictive, and accordingly, the present invention is not limited to the above description, but may be modified within the scope of the appended claims and their equivalents.

## Claims

1. A chromatographic inspection apparatus, comprising:
a main body;
a cartridge tray on which an immunochromatographic test cartridge is seated, and which is provided so as to be accommodated in the main body or to be withdrawn to the outside of the main body;
a tray driving unit for moving the cartridge tray;
an image sensing unit for recognizing a code expression provided on a surface of the cartridge or excitation light generated from the cartridge;
a first light source illuminating the code expression provided on the surface of the cartridge;
a second light source illuminating a window of the cartridge to generate the excitation light in the cartridge; and
a control unit for controlling the tray driving unit, the first light source and the second light source, and processing information acquired from the image sensing unit.

2. The chromatographic inspection apparatus of claim 1, wherein the first light source emits light in a wavelength band different from that of the second light source.

3. The chromatographic inspection apparatus of claim 1, wherein the image sensing unit comprises:
an image sensor for sensing incident light; and
a filter for blocking light other than the wavelength band of the excitation light from being incident on the image sensor.

4. The chromatographic inspection apparatus of claim 3, wherein the first light source emits light in a wavelength band that passes through the filter.

5. The chromatographic inspection apparatus of claim 1, wherein the second light source is installed on both sides of the image sensor and irradiates obliquely from both sides of the window of the cartridge.

6. The chromatographic inspection apparatus of claim 1, further comprising a fixing part for elastically fixing the seated cartridge.

7. The chromatographic inspection apparatus of claim 6, wherein the fixing part comprises:
a ball plunger provided in the cartridge tray and elastically supported, and elastically inserted into a fixing groove formed in the cartridge.

8. The chromatographic inspection apparatus of claim 1, wherein a calibration window, which is formed in the cartridge tray and through which the light of the second light source passes, is further formed.

9. The chromatographic inspection apparatus of claim 1, further comprising a position sensor for recognizing the position of the cartridge.

10. The chromatographic inspection apparatus of claim 9, wherein the position sensor recognizes a position where the code expression of the cartridge is located below the image sensor and a position where the window of the cartridge is located below the image sensor.

11. The chromatographic inspection apparatus of claim 1, further comprising a cartridge recognition sensor provided in the cartridge tray to recognize whether the cartridge is seated.

12. A method for controlling a chromatographic inspection apparatus, comprising:
a cartridge seating step for seating a cartridge in a cartridge tray;
a code expression recognizing step for recognizing a code expression of the cartridge seated in the cartridge tray;
a blood injection step for dropping a sample into the cartridge;
an excitation position moving step for moving the cartridge on which the blood injection has been completed to an excitation position for reading excitation light;
an excitation light source irradiating step for irradiating an excitation light source for emitting excitation light to the cartridge according to the recognition result of the code expression recognizing step; and
an excitation light measuring step for recognizing and measuring excitation light emitted according to the irradiation of the excitation light source.

13. The method of claim 12, wherein the type of marker of the cartridge is recognized according to the recognition result of the code expression recognizing step, and the emission of the excitation light source is controlled to correspond to the type of the marker.

14. The method of claim 12, further comprising a code expression moving step for moving the cartridge seated in the cartridge tray for the recognition of code expression.
